# EUROPEAN PATENT SPECIFICATION

(11) **EP 0 310 413 B1**
(45) Date of publication and mention of the grant of the patent: **16.03.1994**
(21) Application number: 88309093.8
(22) Date of filing: 30.09.1988
(51) Int. Cl.: G01N 33/552, G01N 33/76, G01N 33/74

(54) **Immobilized antibodies**
Immobilisierte Antikörper
Anticorps immobilisés

(30) Priority: 30.09.1987 JP 248923/87
(43) Date of publication of application: 05.04.1989
(73) Proprietor: NIHON CHEMICAL RESEARCH KABUSHIKI KAISHA also known as JCR PHARMACEUTICALS CO., LTD, Kobe Hyogo 658 (JP)
(72) Inventor: Hayashi, Kyozo, 828-1, Iwasaki Gifu Gifu 502 (JP); Kurobe, Masayuki, Gifu Gifu 502 (JP); Nishimuro, Satoshi, Birmingham Alabama 35205 (US); Hiratani, Hajime, Sennan-gun Osaka 599-2 (JP)
(74) Representative: Burford, Anthony Frederick

(56) References cited:
- EP-A- 0 097 364
- EP-A- 0 227 351
- DE-A- 2 042 976
- GB-A- 2 014 727

## Description

This invention relates to immobilized antibodies.

Recent years have shown marked progress in the field of immunoassay using the antigen-antibody reaction. The enzyme immunoassay and radioimmunoassay techniques, which can quantitate trace substances in the living body, are already in wide clinical use for the diagnosis of infectious diseases and endocrine diseases.

Immunoassay utilizes the following characteristic features of the antigen-antibody reaction: said reaction can take place at low concentrations and the resultant antigen-antibody complexes hardly dissociate. Therefore, those immobilized antibodies which are prepared by binding an antibody to a carrier, such as a plastic, a silicone, or a glass, by making good use of the phenomenon of physical adsorption are currently in wide use. However, once they have formed complexes with antigens, the immobilized antibody can not be used repeatedly since treatment of the complexes with an acid or a high concentration of salt solution for the purpose of resolving them for regeneration of the immobilized antibodies will inevitably result not only in dissolution of the antigen-antibody bond but also in dissolution of the antibodies from the carriers. Moreover, in the case of rare antibodies, in particular where the antigens are human-derived substances occurring only in very small amounts, such as human growth hormone, human luteinizing hormone, human glucagon and human gastrin, it is difficult to purify them and also to obtain raw materials containing them. Antibodies obtained by immunizing animals with such substances are very expensive and therefore should desirably be used repeatedly. On the other hand, radioimmunoassay, which must use some or other radiation source, poses problems in the disposal of radioactive waste material.

GB-A-2014727 discloses the preparation of immobilized antibodies by chemically or physically binding the antibody to frosted glass. Reference is made to chemical binding with the aid of a silane coupling agent and, if necessary, a cross-linking agent. The silane coupling agent can be an aminoalkylsilylating agent and specific reference is made to, inter alia, 3-aminopropyltriethoxysilane. Specified cross-linking agents include dimaleimides and maleimido-carboxyl-N-hydroxysuccinimide esters but the preferred cross-linking agent is glutaraldehyde. The exemplified cross-linking agents include 4-(maleimidomethyl)-cyclohexane-1-carboxyl-N-hydroxysuccinimide ester and N,N'-o-phenylenedimaleimide.

DE-A-2042976 (corresponding to US-A-3652761) discloses immobilized antibodies in which the antibody is chemically bound to glass by means of a silane coupling agent. Preferred coupling agents are amino-functional aliphatic silanes and the exemplified silanes include 3-aminopropyltriethoxysilane. No reference is made to the use of cross-linking agents between the antibody and the silane coupling agent.

EP-A-0227351 discloses an immobilized antibody in which a partially reduced form of IgG specific to human proinsulin C-peptide is bound to glass by a hetero-bifunctional cross-linking agent. The glass surface can be aminoalkylsilylated and the specified cross-linking agents include various maleimidocarboxyl-N-hydroxysuccinimide esters including 4-(N-maleimidomethyl)-cyclohexane-1-carboxylic acid N-hydroxysuccinimide ester.

The present inventors made intensive investigations and have succeeded in preparing immobilized antibodies capable of being used repeatedly in enzyme immunoassay. Thus, they succeeded in reducing costs through the repeated use of the immobilized antibodies as well as in reducing the waste discharge, and have now completed the present invention.

The present invention is directed to immobilized antibodies comprising an Fab' antibody fraction covalently bound to a maleimide coupling agent to a glass body surface activated by reaction with an aminoalkylsilylating agent.

The glass body to be used as the immobilizing carrier in practice of the invention may have the form of bead, fiber, disc or test tube. For use in radio- immunoassay, commercially available glass test tubes having a diameter of 5-10 mm and a length of 5-10 mm are particularly preferred because they can be used not only as vessels for the antigen-antibody reaction but also as measurement vessels.

The reaction of an aminoalkylsilylating agent, such as 3-aminopropyltriethoxysilane, with the glass surface results in introduction of an amino group as a result of aminoalkylsilylation of the glass surface.

Usable as the Fab' antibody are fractions obtained from IgG species prepared by immunizing a warm-blooded animal with the antigen to be assayed as well as commercially available antibody IgG species.

Each IgG species is converted to Fab' in advance so that the thiol groups can be exposed. The conversion of IgG to Fab' can be performed in the conventional manner, for example by purifying IgG by salting out and the use of an ion exchange resin, then digesting the same with pepsin and bringing the resulting F(ab')₂ into contact with a reducing agent.

The glass-Fab' binding is performed using a maleimide cross-linking or coupling agent, such as N,N'-o-phenylenedimaleimide, N,N'-o-oxydimethylenedimaleimide, N-(4-carboxyphenylmethyl)maleimide or N-succinimidyl 4-(maleimidomethyl)-cyclohexane-1-carboxylate (SMCC). A particularly preferred crosslinking agent is SMCC.

It is preferable that the glass surface be first reacted with the crosslinking agent and then reacted with the antibody Fab'.

The reactions can be conducted under mild conditions, for example in a phosphate buffer (pH) at a temperature of about 37°C for about 1 hour.

There is thus obtained an immobilized antibody with the antibody covalently bound to glass.

The immobilized antibody obtained in the above manner can be used in the sandwich technique of immunoassay by reacting said immobilized antibody first with an antigen and then with an enzyme or a radio-labeled antigen, then separating the solid phase from the liquid phase, and measuring either one of the phases for enzyme quantity or radioactivity, and in the competitive immunoassay technique by reacting an enzyme or a radio-labeled antigen on one hand and an unlabeled antigen on the other competitively with said immobilized antibody, then separating the solid phase from the liquid phase and measuring either one of the phases for enzyme quantity or radioactivity.

In both immunoassay techniques, the immobilized antibody can be used repeatedly many times after mere washing with a dilute acid, preferably an aqueous solution of acetic acid, following completion of each assay.

In accordance with the invention, any antibody IgG species can be used irrespective of the kind of antigen.

The following examples illustrate the invention in further detail.

### Example 1

### (1) Preparation of anti-human epidermal growth factor antiserum

Human epidermal growth factor (hereinafter abbreviated as hEGF) was prepared from human urine by the method described in Japanese Kokai Tokkyo Koho 58-219124 and U. S. Patent 4,528,186. hEGF (0.5 mg) was dissolved in 0.5 ml of physiological saline, an equal volume of complete Freund's adjuvant was added and, after mixing for emulsification, the mixture emulsion was injected into a rabbit at several sites on the back, followed by three subcutaneous injections of the same emulsion in the same dose at 2-week intervals. Ten days after the final immunization, the whole blood was collected, allowed to stand at room temperature for coagulation and then centrifuged at 3,000 rpm for 10 minutes to give an anti-hEGF antibody-containing antiserum.

### (2) Preparation of Fab'

The anti-hEGF antibody-containing antiserum obtained in (1) was applied to a column of Protein A-Sepharose® CL-4B (Pharmacia Japan, Tokyo) to thereby cause adsorption of IgG. Then the column was washed well with 0.1 M phosphate buffer (pH 8.0) containing 0.15 M NaCl and then IgG was eluted from the column with 1 M acetic acid.

A 10 mg portion of this IgG was digested with pepsin (Boehringer Mannheim-Yamanouchi Co., Ltd., Tokyo) in 0.1 M acetate buffer (pH 4.0). The digest was purified using a column of Sephadex® G-100 (Pharmacia Japan, Tokyo) and then passed through a column of Protein A-Sepharose® CL-4B for the removal of undigested antibody. The thus-obtained F(ab')₂ fragment was further reduced with 2-mercaptoethanolamine and the reduction product was purified on a column of Sephadex® G-25 (Pharmacia, Sweden) to give an Fab' fragment.

### (3) Preparation of immobilized antibody

Glass-made test tubes (0.8 cm in diameter, 7.5 cm in length; Corning Glass Works, New York) were dried at 180°C for 2 hours. Then, 0.1 ml of a 2% (w/v) solution of 3-aminopropyltriethoxysilane (Aldrich Chemical Co., Inc., U.S.A.) in acetone was added to each test tube. The excess acetone solution was removed, and the test tube was washed repeatedly with acetone and then allowed to stand at 60°C for 2 hours.

To the thus-aminoalkylsilylated glass test tube was added 0.1 ml of 0.1 M phosphate buffer (pH 6.0) containing N-succinimidyl 4-(N-maleimidomethyl)cyclohexane-1-carboxylate (Pierce Chemical Co., U.S.A.) and N,N-dimethylformamide in concentrations of 7.2 mg and 0.12 ml, respectively, per 6 ml. After incubation at 37°C for 60 minutes, the test tube was washed with three 0.5 ml portions of 0.1 M phosphate buffer and, immediately thereafter, 0.1 ml of 0.1 M phosphate buffer containing the Fab' fragment was added and incubation was carried out at 37°C for 60 minutes, followed by washing with three portions of 0.1 M phosphate buffer containing 0.1% bovine serum albumin, 0.3 M NaCl, 0.1% NaN₃ and 1 mM MgCl₂. Thus was prepared a glass body with anti-hEGF Fab' immobilized thereon. The quantity of Fab' bound per test tube as calculated on the basis of the reduction in absorbance at 280 nm of the Fab' solution submitted to the reaction was 4.9 µg.

### (4) Preparation of peroxidase-coupled Fab'

Peroxidase (2 mg; Nordic Immunolgica Laboratories, Netherlands) was dissolved in 0.3 ml of 0.1 M phosphate buffer. To the solution was added a 0.02 ml N,N-dimethyl-formamide solution of 1.6 mg of N-succinimidyl 4-(N-maleimidomethyl)cyclohexane-1-carboxylate (Pierce Chemical Co., U.S.A.). The mixture was incubated at 60°C for 30 minutes. Then, after removal of the resultant precipitate by centrifugation, the reaction mixture was purified on a Sephadex® G-25 column to give a maleimidated peroxidase solution. Thereto was added 2.86 mg of the anti-hEGF antibody Fab' fragment, and the reaction was allowed to proceed at 4°C for 30 minutes. The reaction mixture was purified on a column of Ultrogel® Ac44 (Pharmacia Japan, Tokyo) equilibrated with 0.1 M phosphate buffer (pH 6.0) to give peroxidase-coupled Fab' fragment.

### (5) Assay of hEGF

Purified hEGF was dissolved in concentrations of 10 pg to 1,000 pg per milliliter in phosphate buffer (pH 7.0) containing 0.1 M NaCl, 1 mM MgCl₂ and 0.1% bovine serum albumin. The solutions thus prepared were used as standard hEGF solutions. A 0.1 ml portion of each standard solution was added to a glass test tube prepared in (3). After standing overnight at 4°C, each test tube was washed with 0.1 M phosphate buffer (pH 7.0) containing 0.1% bovine serum albumin. Then, 0.2 ml of a buffer containing 4.3 ng of the peroxidase-coupled Fab' prepared in (4) was added and incubation was further conducted at room temperature for 6 hours. After washing well with a buffer, 0.1 ml of 0.6% (w/v) 3-(p-hydroxyphenyl)propionic acid (Wako Junyaku Co., Ltd., Tokyo) was added. After 5 minutes, 0.1 ml of 0.015% aqueous hydrogen peroxide was added. After 1 hour of incubation at 25°C, 0.2 ml of 0.1 M glycine-NaOH buffer (pH 10.3) was added to thereby terminate the reaction, and fluorescence measurement was conducted on a fluorophotometer (Hitachi Ltd., Tokyo; model 650-60) at an excitation wavelength of 320 nm and a measurement wavelength of 405 nm.

After completion of the above assay procedure, the antibody-coupled glass test tubes were washed with three portions of 0.1% aqueous acetic acid and then with 0.1 M phosphate buffer (pH 7.0) and again used for assaying hEGF.

The above series of operations was repeated ten times in all. Comparison of the thus-obtained working curves for hEGF failed to reveal any significant differences among them.

### Example 2

### (1) Preparation of anti-human chorionic gonadotropin antiserum

Human chorionic gonadotropin (hereinafter, hCG) was prepared by the method described in Japanese Kokai Tokkyo Koho 58-99421 and United States Patent 4,665,161. The procedure of Example 1(1) was repeated using said hCG (0.5 mg) to obtain anti-hCG antibody-containing antiserum.

### (2) Preparation of Fab'

The procedure of Example 1(2) was repeated using the anti-hCG antibody-containing antiserum obtained in (1) to give an Fab' fragment.

### (3) Preparation of immobilized antibody

The procedure of Example 1(3) was repeated using the Fab' fragment obtained in (2) to obtain a glass body with anti-hCG Fab' immobilized thereon. The quantity of Fab' bound per test tube as calculated on the basis of the reduction in absorbance at 280 mm of the Fab' solution submitted to the reaction was 5.2 µg.

### (4) Preparation of peroxidase-coupled Fab'

The procedure of Example 1(4) was repeated using the anti-hCG antibody Fab' fragment obtained in (2) to give peroxidase-coupled Fab' fragment.

### (5) Assay of hCG

The procedure of Example 1(5) was repeated using purified hCG dissolved in concentrations of 5 mIU to 1,000 mIU per milliliter in the phosphate buffer to obtain standard hCG solutions. Comparison of the working curves for hCG failed to reveal any significant differences among them.

### Example 3

### (1) Preparation of anti-adrenocorticotropic hormone (anti-ACTH) Fab'

The procedure of Example 1(2) was repeated using a commercial anti-ACTH antibody-containing antiserum (Seikagaku Kogyo Co , Ltd., Japan) to give an Fab' fragment.

### (2) Preparation of immobilized antibody

The procedure of Example 1(3) was repeated using the Fab' fragment obtained in (1) to obtain a glass body with anti-ACTH Fab' immobilized thereon. The quantity of Fab' bound per test tube as calculated on the basis of the reduction in absorbance at 280 nm of the Fab' solution submitted to the reaction was 5.1 µg.

### (3) Assay of ACTH

The ¹²⁵I-ACTH and standard ACTH used were those contained in the ACTH I-125 kit (The Green Cross Corp., Japan). The standard ACTH was diluted to 50 to 800 pg/ml.

To each of the glass test tubes prepared in (2) was added 0.7 ml of 0.02 M veronal buffer, and, then, 0.1 ml of the ¹²⁵I-ACTH solution and 0.1 ml of a standard ACTH solution were added in that order. After 48 hours of incubation at 4°C, the test tube was washed three times with veronal buffer and the radioactivity adsorbed on the glass test tube was measured with a gamma-counter (Beckman model gamma 5500). The glass test tubes after assay were washed with three portions of 0.1% aqueous acetic acid and then with 0.1 M phosphate buffer (pH 7.0) and again used for the assay of ACTH. The above series of operations was repeated ten times in all. Comparison of the thus-obtained working curves for ACTH failed to reveal any significant difference.

## Claims

1. An immobilized antibody which comprises an Fab' antibody fraction covalently bound by a maleimide coupling agent to a glass body surface activated by reaction with an aminoalkylsilylating agent.

2. An immobilized antibody as claimed in Claim 1, wherein the aminoalkylsilylating agent is 3-aminopropyltriethoxysilane.

3. An immobilized antibody as claimed in Claim 1 or Claim 2, wherein the maleimide is N,N'-o-phenylene dimaleimide, N,N'-o-oxy dimethylene dimaleimide, N-(4-carboxyphenylmethyl) maleimide or N-succinimidyl-4-maleimideomethyl-cyclohexane-1-carboxylate.

4. An immobilized antibody as claimed in any one of the preceding claims, wherein the Fab' fraction is derived from anti-human growth factor antiserum, anti-human chorionic gonadotropin antiserum or anti-adrenocorticotropic hormone antiserum.

5. An immobilized antibody as claimed in any one of the preceding claims, wherein the glass surface is at least a portion of the inner surface of a test tube.

6. A method of preparing an immobilized antibody as claimed in Claim 1, comprising aminoalkylsilylating a glass surface by reaction with an aminoalkylsilylating agent; reacting the aminoalkylsilylated surface with a maleimide cross-linking agent; and reacting the resultant treated surface with a Fab' antibody fraction.

7. The use of an immobilized antibody as claimed in any one of the preceding claims in an immunoassay.

8. A use as claimed in Claim 7, wherein the immobilized antibody has been regenerated from previous use by washing with a dilute acid.

9. A use as claimed in Claim 8, wherein the dilute acid is aqueous acetic acid.

## Patentansprüche

1. Immobilisierter Antikörper, umfassend eine Fab'-Antikörperfraktion, die durch ein Maleimid-Kopplungsmittel kovalent an eine Glaskörperoberfläche gebunden ist, welche durch Reaktion mit einem Aminoalkylsilylierungsmittel aktiviert ist.

2. Immobilisierter Antikörper nach Anspruch 1, bei dem das Aminoalkylsilylierungsmittel 3-Aminopropyltriethoxysilan ist.

3. Immobilisierter Antikörper nach den Ansprüchen 1 oder 2, bei dem das Maleimid N,N'-o-Phenylendimaleimid, N,N'-o-Oxydimethylendimaleimid, N-(4-Carboxyphenylmethyl)-maleimid oder N-Succinimidyl-4-maleimideomethyl-cylcohexan-1-carboxylat ist.

4. Immobilisierter Antikörper nach einem der vorhergehenden Ansprüche, bei dem die Fab'-Fraktion abgeleitet ist vom anti-humanen Wachstumsfaktor-Antiserum, anti-humanen Choriongonadotropin-Antiserum oder anti-adrenokortikotropen Hormon-Antiserum.

5. Immobilisierter Antikörper nach einem der vorhergehenden Ansprüche, bei dem die Glasoberfläche mindestens ein Teil der inneren Oberfläche eines Teströhrchens ist.

6. Verfahren zur Herstellung eines immobilisierten Antikörpers gemäß Anspruch 1, umfassend das Aminoalkylsilylieren einer Glasoberfläche durch Reaktion mit einem Aminoalkylsilylierungsmittel, das Umsetzen der aminoalkylsilylierten Oberfläche mit einem Maleimid-Vernetzungsmittel, und das Umsetzen der resultierenden behandelten Oberfläche mit einer Fab'-Antikörperfraktion.

7. Verwendung eines immobilisierten Antikörpers gemäß einem der vorhergehenden Ansprüche in einem Immunoassay.

8. Verwendung nach Anspruch 7, bei der der immobilisierte Antikörper aus einer vorherigen Verwendung durch Waschen mit einer verdünnten Säure regeneriert worden ist.

9. Verwendung nach Anspruch 8, bei der die verdünnte Säure wäßrige Essigsäure ist.

## Revendications

1. Anticorps immobilisé qui comprend une fraction Fab de l'anticoprs en liaison covalente, par l'intermédiaire d'un agent de couplage maléimide, avec la surface d'une pièce de verre, activée par réaction avec un agent d'aminoalkylsilylation.

2. Anticorps immobilisé selon la revendication 1, dans lequel l'agent d'aminoalkylsilylation est le 3-aminopropyltriéthoxysilane.

3. Anticorps immobilisé selon la revendication 1 ou 2, dans lequel le maléimide est le N,N'-o-phénylènedimaléimide, le N,N'-o-oxydiméthylènedimaléimide, le N-(4-carboxyphénylméthyl)maléimide ou le N-succinimidyl-4-maléimidométhylcyclohexane-1-carboxylate.

4. Anticorps immobilisé selon l'une quelconque des revendications précédentes, dans lequel la fraction Fab dérive de l'antisérum anti-facteur de croissance humain, de l'antisérum anti-gonadotropine chorionique humaine ou de l'antisérum anti-hormone adrénocorticotropique.

5. Anticorps immobilisé selon l'une quelconque des revendications précédentes, dans lequel la surface de verre est au moins une partie de la surface intérieure d'un tube à essais.

6. Procédé de préparation d'un anticorps immobilisé selon la revendication 1, comprenant l'aminoalkylsilylation d'une surface de verre par réaction avec un agent d'aminoalkylsilylation; la réaction de la surface aminoalkylsilylée avec un agent de réticulation maléimide; et la réaction de la surface traitée qui en résulte avec une fraction Fab de l'anticorps.

7. Utilisation d'un anticorps immobilisé selon l'une quelconque des revendications précédentes dans un essai immunologique.

8. Utilisation selon la revendication 7, dans laquelle l'anticorps immobilisé a été régénéré à partir d'une utilisation précédente par lavage avec un acide dilué.

9. Utilisation selon la revendication 8, dans laquelle l'acide dilué est l'acide acétique aqueux.
